(19) Europäisches Patentamt
European Patent Office
Office européen des brevets

(11) EP 2 106 819 A1

(12) EUROPEAN PATENT APPLICATION

(43) Date of publication:
07.10.2009 Bulletin 2009/41

(51) Int Cl.:
A61M 16/00 (2006.01)     A61M 16/20 (2006.01)

(21) Application number: 08006506.3

(22) Date of filing: 31.03.2008

(84) Designated Contracting States:
AT BE BG CH CY CZ DE DK EE ES FI FR GB GR
HR HU IE IS IT LI LT LU LV MC MT NL NO PL PT
RO SE SI SK TR
Designated Extension States:
AL BA MK RS

(71) Applicant: Linde AG
80331 München (DE)

(72) Inventors:
• Romako, Christian
A-3382 Loosdorf (AT)
• Köhle, Christian
A-2120 Wolkersdorf im Weinviertel (AT)

(74) Representative: Schüssler, Andrea et al
Kanzlei Huber & Schüssler
Truderinger Strasse 246
81825 München (DE)

(54) **Pressure support ventilation with a passive PEEP valve**

(57)     The present invention provides a ventilator suitable for assisting ventilation of a mammalian subject, and particularly a human patient: this ventilator employs an exhalation valve, and a simple, passive PEEP valve. During the ventilator's exhalation phase the respiratory gas exits through this passive PEEP valve, which operates independently of ventilator functions and maintains a predetermined minimum gas pressure within the ventilation system: **i.e.** within the ventilator's inhalation path and exhalation path and at an external interface. The external interface provides respiratory gas from the ventilator to the mammalian subject by known means: for example a Y-piece connecting the inhalation and exhalation paths to a breathing mask. Moreover, the invention provides components for monitoring the pressure and flow of respiratory gas so that the ventilator detects and supports the mammalian subject's spontaneous inhalation and/or exhalation.

Figure 5

**Description**

**Field of the Invention**

The present invention relates generally to the field of physiological respiration and particularly to the field of non-invasive ventilators capable of assisting a mammalian subject to breath. The non-invasive intermittent ventilator of the present invention provides pressure support ventilation (PSV) to a patient: it supplies respiratory gas to a patient during a controlled inhalation phase and employs a passive PEEP valve to maintain a selected minimum pressure during a controlled exhalation phase. The invention provides means for the ventilator to detect spontaneous inhalation and/or exhalation by the patient, and to adapt the ventilator's controlled inhalation and exhalation cycle to support such spontaneous inhalation and exhalation.

**Background to the Invention**

It is accepted medical and veterinary practice to use mechanical ventilatory therapy to treat patients suffering from respiratory disorders. It is well known that the use of mechanical ventilatory techniques for an extended period may produce detrimental side effects such as atrophy of respiratory muscles, decreased lung volume and decreased lung compliance (pulmonary volume change per unit pressure change; a measure of the elasticity of the lungs), often resulting in an inability of the patient to return to breathing without artificial assistance.

Non-invasive ventilators are known in the art which provide continuous positive airway pressure (CPAP) through application of a constant pressure to the patient's airways throughout respiration. The dosing device must therefore provide the maximum flow demanded by the patient during the whole breathing cycle, with correspondingly high gas consumption. Such ventilators are often used to treat obstructive sleep apnea, Cheyne-Stokes respiration and other breathing disorders commonly associated with congestive heart failure: among other effects they raise the pressure in the chest cavity surrounding the heart, allowing cardiac output to increase (*see* Hill, US Patent Application Publication No. 2002/0088465 A1). Bilevel CPAP machines are known which ventilate the patient with a combination of a basal level of CPAP and, during the inhalation phase, additional pressure support (for example Zdrojkowski et al., US Patent Application Publication No. 2002/0023645 A1).

Non-invasive ventilators are also known which provide mechanical ventilatory support in modes termed assist-control ventilation (ACV) or synchronised intermittent mandatory ventilation (SIMV). Both ACV and SIMV are 'volume assist' ventilation modes: the patient's spontaneous inhalatory efforts are detected by the ventilator. The ventilator responds in ACV mode by delivering a pre-selected tidal volume and flow rate of respiratory gas. In SIMV mode the ventilator responds with respiratory assistance only after detecting a predetermined number of spontaneous inhalatory efforts by the patient, thereby assisting in weaning the patient from the ventilator.

To reduce the patient's dependence upon the ventilator, encourage spontaneous respiration, accelerate weaning of the patient from the ventilator and to minimise patient discomfort, the ventilator must guarantee that the patient receives an adequate supply of respiratory gas while simultaneously allowing the patient as normal a respiration pattern as possible. As disclosed in the comparative studies of McIntyre, a further mode of mechanical ventilatory support, pressure support ventilation, may achieve these aims more effectively than either ACV or SIMV.

Non-invasive pressure support ventilation (PSV) is a pressure-limited, flow-cycled mode of assisted ventilation in which the pressure support exceeds PEEP. As described by Hotchkiss *et al.*, each inhalation is supported by the supply of respiratory gas at a constant selected pressure to the patient, resulting in delivery of respiratory gas to the patient at a flow rate that decreases from a maximum value during the course of inhalation. Inhalation terminates when measured inspiratory flow falls below a set value or a selected fraction of the peak flow rate. A lower pressure exhalation phase then commences in which a positive airway pressure (CPAP) may be maintained (Hotchkiss et al., 2002, Am J Respir Crit Care Med 165, 47-53).

Mammals must maintain within their lungs a positive exhalation end pressure (PEEP) above ambient atmospheric pressure in order to prevent collapse of the lower airways and the alveoli, and other physiological dysfunctions such as obstruction of the upper airways due to compression resulting from high transmural pressures during forced expiration. The PEEP increases the pressure in the airways and lowers the transmural pressure by a significant value, thereby enhancing ventilation and reducing dynamic hyperinflation. Physiological factors such as flow resistance in the lungs and airways may increase PEEP, and in a patient receiving respiratory assistance it may also be influenced by apparatus-related factors, for example the ratio of the duration of inspiration *versus* expiration, or the ratio of respiratory rate to tidal volume.

However, an increase in PEEP increases the functional residual capacity of the lungs - the quantity of gas remaining in the lungs after passive exhalation - and decreases the available respiratory tidal volume. Non-invasive respiratory assistance provided to human or animal patients must therefore ensure that a PEEP is maintained which is appropriate for optimal protection of physiological tissues while avoiding interference with either the natural or the artificially assisted respiratory activities.

Non-invasive ventilators are known in the art which combine assisted ventilation with the maintenance of respiratory gas pressure at or above a selected PEEP pressure at all stages of the inhalation and exhalation cycle. For example, Schwanbom *et al*. disclose in US Patent 4,211,221 a ventilator which operates in either CPAP or SIMV mode; the ventilator employs a 'respiratory gas measuring station' which monitors the passage of respiratory gas passing to and from a patient during inhalation and exhalation respectively. Exhaled gas passes through an exhalation control which comprises a check valve, an exhalation gas meter and an exhalation valve venting into the atmosphere. The patient may inhale and exhale spontaneously, interrupting the timed inhalation/exhalation cycle of the SIMV mode: in this case the timed cycle re-starts in synchrony with the end of spontaneous expiration.

In SIMV mode the apparatus disclosed by US 4,211,221 employs a passive 'PEEP valve' which interacts directly with the operation of the pneumatically controlled exhalation valve: this 'PEEP valve' is located in a distinct pneumatic pressure control line which governs the maximum closing force imposed by the exhalation valve during the exhalation phase. Consequently, during the exhalation phase the exhalation valve maintains an adjustable 'exhalation resistance' between the exhalation path and the atmosphere. During the inhalation phase the exhalation valve functions as a closing mechanism.

In European Patent Application 0 811 394 A1, Christian discloses a ventilator capable of operating in a number of modes, including pressure support mode, in which the expiration section comprises an expiration pressure gauge and an exhalation valve. In this ventilator a minimum pressure is maintained as a selected PEEP by a microprocessor which controls operation of the exhalation valve, using signals of gas flow and pressure recorded in the exhalation path 8.

The exhalation and PEEP valves in the ventilators described in the prior art are complex: they receive and/or deliver signals to a controller, for example a microprocessor. Additionally, these controlled exhalation and PEEP valves are expensive so that they must be reused: because gas respired by the patient passes through the valves they must be sterilised reliably before and/or after use with the ventilator. Such sterilisation would routinely be performed by, for example, autoclaving the valves with superheated steam under pressure, a process that is likely to significantly reduce the usable life-span of such complicated mechanical or electromechanical components.

The art therefore lacks a means of providing assisted support ventilation, for example non-invasive pressure support ventilation, in which the pressure of respiratory gas at or above a selected PEEP can be maintained throughout all phases of a patient's respiration by means of a simple exhaust valve, and a simple PEEP valve which operates independently of ventilator functions.

A factor influencing this need in the art is that simple mechanical PEEP valves are known to open and close at pressures that may vary as a result of numerous factors, such as temperature, the humidity of atmospheric and respired gas, material imperfections and mechanical wear and fatigue. Such fluctuations in operating characteristics may cause the gas pressure that the ventilator maintains at end of the expiration phase (the established PEEP) to vary over a range which is significantly greater than the patient-induced differences that a responsive, modem ventilator should recognise as a trigger for modulating the inhalation or exhalation phases. These factors have dissuaded those of skill in the art from using simple PEEP valves with respiratory ventilators for controlling minimum respired gas pressure and maintaining PEEP.

### Summary of the Invention

The present invention provides a ventilator suitable for assisting ventilation of a mammalian subject, and particularly a human patient: this ventilator employs an exhalation valve, and a simple, passive PEEP valve. During the ventilator's exhalation phase the respiratory gas exits through this passive PEEP valve, which operates independently of ventilator functions and maintains a pre-determined minimum gas pressure within the ventilation system: *i.e.* within the ventilator's inhalation path and exhalation path and at an external interface. The external interface provides respiratory gas from the ventilator to the mammalian subject by known means: for example a Y-piece connecting the inhalation and exhalation paths to a breathing mask. Moreover, the apparatus and methods of the invention provide components for monitoring the pressure and flow of respiratory gas so that the ventilator detects and supports the mammalian subject's spontaneous inhalation and/or exhalation.

A first embodiment of the invention provides a ventilator suitable for assisting respiration in a mammal, said ventilator comprising a control unit, a gas flow controller, an inhalation path leading respiratory gas from said gas flow controller to an external interface, an exhalation path leading respiratory gas from the external interface to the atmosphere, and a pressure sensor for detecting the pressure of respiratory gas at the external interface; wherein said exhalation path comprises a flow sensor, an exhalation valve and a passive PEEP valve; wherein said ventilator is embodied to operate alternately in a ventilator inhalation phase and in a ventilator exhalation phase; and wherein in each said ventilator exhalation phase said passive PEEP valve maintains a minimum pressure in the exhalation path independently of the operation of the control unit, and said control unit is embodied to: control the exhalation valve open and the supply of respiratory gas from said gas flow controller closed, measure said minimum pressure by means of the pressure sensor when the absolute gas flow rate detected by the flow sensor in the exhalation path achieves zero flow, or a pre-determined low flow rate, calculate a trigger level for the pressure at which the control unit triggers a ventilator inhalation phase, wherein said trigger level is derived from said minimum pressure, and determine when the pressure detected by means of the pressure sensor is less than or equal to the calculated trigger level, and trigger a ventilator inhalation phase by closing the exhalation valve and supplying respiratory gas to the external interface via the inhalation path with the gas flow controller.

A further embodiment of the invention provides a method of controlling the operation of a ventilator employing a passive PEEP valve, said ventilator 1 comprising a control unit, a gas flow controller, an inhalation path leading respiratory gas from said gas flow controller to an external interface, an exhalation path leading respiratory gas from said external interface to the atmosphere, and a pressure sensor for detecting the pressure of respiratory gas at said external interface; wherein said exhalation path comprises a flow sensor, an exhalation valve and a passive PEEP valve; wherein said method comprises operating said ventilator alternately in a ventilator inhalation phase and in a ventilator exhalation phase; wherein in each said ventilator exhalation phase the gas flow controller supplies no gas and the exhalation valve is open; wherein in each said ventilator exhalation phase while the patient is exhaling said passive PEEP valve functions independently of the control unit and exerts a resistance which ensures that the respiratory gas pressure in the exhalation path does not fall below a PEEP valve pressure, and wherein, in each said ventilator exhalation phase, said method comprises the steps of: measuring by means of the pressure sensor a pressure $PEEP_{low\_flow}$ which is the pressure of respiratory gas in the exhalation path when the absolute gas flow rate detected by the flow sensor in the exhalation path attains a pre-determined low flow rate (which is ideally a zero flow rate); calculating a trigger level for triggering an inhalation phase, wherein said trigger level is derived from said pressure $PEEP_{low\_flow}$; determining when the pressure detected by means of the pressure sensor is less than or equal to the trigger level; and triggering a ventilator inhalation phase by closing the exhalation valve and supplying respiratory gas to the external interface via the inhalation path with the gas flow controller.

The invention provides a solution to the technical difficulties experienced when a passive PEEP valve, for example a simple, mechanical, passive PEEP valve, is used for maintaining a pre-defined gas pressure in an apparatus providing artificial ventilation assistance to a mammalian subject. Although the absolute pressure 'PEEP valve pressure' at which such a passive PEEP valve closes may vary due to the influence of several external parameters, the method of the present invention refers to pre-determined criteria in order to determine the pressure $PEEP_{low\_flow}$ achieved during each exhalation phase. In each exhalation phase the ventilator's control unit calculates the trigger level which is set below the pre-defined pressure $PEEP_{low\_flow}$ which the control unit determines for each exhalation phase from pressure and flow data monitored during the individual exhalation phases.

When the control unit determines that the pressure within the exhalation path has dropped to a value less than or equal to the trigger level the current ventilator exhalation phase is terminated and a ventilator inhalation phase is initiated. By implementing this method of determining the pressure at which an individual exhalation phase should be terminated a ventilator comprising a simple, mechanical, passive PEEP valve may be used to assist a ventilated subject's spontaneous attempts to breathe, with extremely sensitive detection of the subject's initiation of inhalation. Such a ventilator may therefore provide a correspondingly rapid and timely response to the subject's attempt to inhale by terminating the controlled ventilator exhalation phase, and initiating a controlled ventilator inhalation phase in which the subject is provided with an appropriate supply of respiratory gas.

The solution provided by the present invention enables a ventilator to maintain these characteristics even where the minimum pressure maintained by the simple passive PEEP valve fluctuates between individual exhalation phases by substantially more than the pressure difference used to calculate the trigger level which signals termination of the ventilator's exhalation phase and initiation of the next ventilator inhalation phase.

Consequently the present invention provides a number of advantages over ventilators known in the art and methods of using the known ventilators. These advantages include, but are not limited to the following:

simplicity of selection:

- a passive PEEP valve operating at a nominal minimum pressure value may be selected for the passive PEEP valve from a series of such valves providing nominal minimum pressure values covering a standard pressure range;

ease of use:

- the passive PEEP valve employed by the ventilator 1 for controlling minimum respired gas pressure and maintaining a PEEP valve pressure may be a simple, mechanical, disposable product and/or a modular 'plug-in' component of the ventilator;

economic considerations:

- the passive PEEP valve may be manufactured by third parties, *e.g.* OEM suppliers, encouraging competition and cheaper supply of these valves as a commodity: the method of the invention compensates for greater operating tolerances that may be associated with cheaply produced passive PEEP valves without compromising effectiveness or sensitivity of ventilator operation. The exhalation valve may also be a disposable component. The flow sensor for determining the respiratory gas flow rate may also be a cheap, simple component, *e.g.* a zero point flow sensor, because the sensor is only required to provide accurate measurements over a limited range.

straightforward sterilization:

- the passive PEEP valve may be sterilized before and/or after use by any common sterilization method which does not damage its particular mechanism; including but not limited to gamma radiation, autoclaving, gas sterilization, dry heat;
- as an example, one effective means of sterilization of the passive PEEP valve is the use of ionizing radiation to sterilize the commercially produced materials before contact with the patient (as with other sterilized medical supplies) coupled with disposal and incineration of the passive PEEP valve after use with the ventilator for ventilating a particular patient;
- commonly, the passive PEEP valve will be a cheap, single-use, disposable item that is sterilized by the manufacturer before or after being packaged;

straightforward operation:

- although the ventilator of the invention is able to employ a simple PEEP valve which may result in fluctuations of PEEP valve pressure from one exhalation phase to another, the control unit automatically calculates an appropriate pressure at which to terminate the ventilator exhalation phase and initiate the ventilator inhalation phase.. Such fluctuations may arise from the ventilation process itself (Hotchkiss *et al*., 2002) from changes in humidity in the expired air, from mechanical irregularities or material imperfections;

**Brief Description of the Figures**

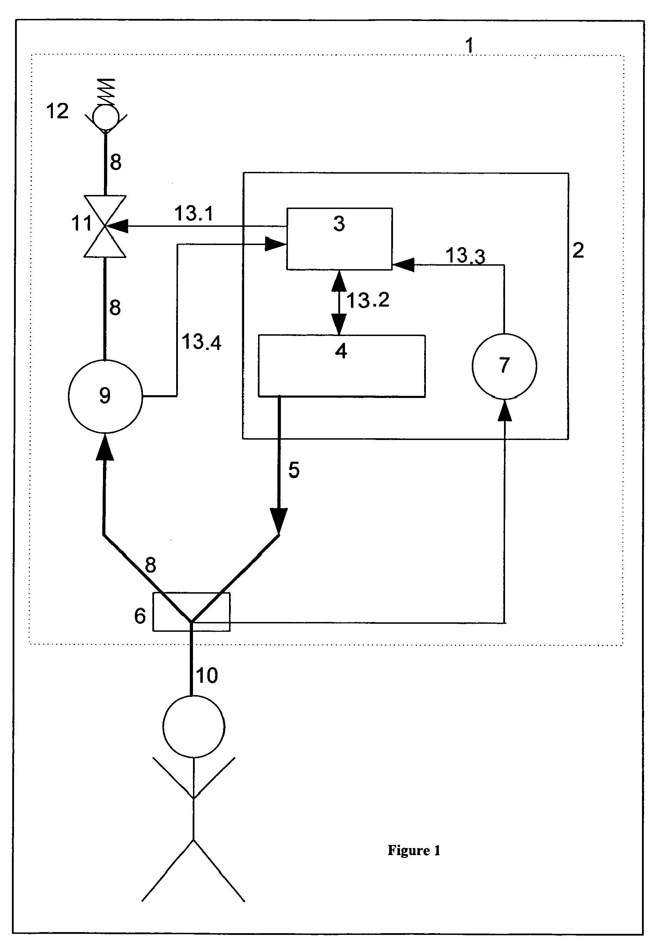

FIGURE 1: Ventilator of the invention.

FIGURE 2: Pressure support ventilation (PSV) showing the variation of gas pressure and gas flow at the external interface 6, over time:

Fig. 2A: Gas pressure during ventilation cycle.

Fig. 2B: Pressure support ventilation (PSV); variation of gas flow through the external interface 6 during ventilation cycle.

FIGURE 3: Expanded view of gas pressure at the end of expiration phase showing effect of estimated PEEP upon trigger level.

FIGURE 4: Diagram of the pressure *versus* flow rate: PEEP valve 7.5 cm $H_2O$ (sample, Vendor 1).

FIGURE 5: Diagram of the pressure *versus* flow rate: PEEP valve 7.5 cm $H_2O$ (sample, Vendor 2).

## Detailed Description of the Invention

THE VENTILATOR OF THE INVENTION

The ventilator 1 of the invention comprises a control unit 2. The control unit 2 comprises a microprocessor 3 and a gas flow controller 4.

The gas flow controller 4 of the control unit 2 is connected via an inhalation path 5 to an external interface 6. The inhalation path 5 may be constructed as a tube.

A pressure sensor 7 is connected to the inhalation path 5, the external interface 6 and/or an exhalation path 8 and may be integrated into the control unit 2.

The external interface 6 is connected via the exhalation path 8 to a flow sensor 9. The exhalation path 8 may be constructed as a tube. Furthermore the external interface 6 comprises an external connection 10. This external connection 10 may be connected to a face mask (not shown).

The external interface 6 may be constructed as a Y-piece which connects the inhalation path 5, the external connection 10 and the exhalation path 8. The Y-piece may be constructed as a tube.

From the flow sensor 9 the exhalation path 8 leads to an exhalation valve 11.

The exhalation valve 11 is connected to a passive PEEP valve 12 via the exhalation path 8. The passive PEEP valve 12 may be connected directly to the outlet of the exhalation valve 11. At a certain pressure level the passive PEEP valve 12 closes the exhalation path 8. In the following this pressure level is termed "PEEP valve pressure".

The exhalation valve 11, the gas flow controller 4, the pressure sensor 7 and the flow sensor 9 are connected to the microprocessor 3 of the control unit 2 via a switch data line 13.1, a control data line 13.2, a pressure data line 13.3 and a flow data line 13.4.

The passive PEEP valve 12 preferably comprises a spring loaded membrane (not shown) by means of which the valve closes the exhalation path 8.

The passive PEEP valve 12 may comprise alternative pressure valve operating means to the spring-loaded membrane, although the valve must restrict the gas flow rate progressively as gas pressure decreases towards the pressure at which the valve closes. The passive PEEP valve 12 is preferably a simple and cheap mechanically controlled component and is preferably disposable. Thus the PEEP valve pressure is subject to certain tolerances.

The PEEP valve 12 would prevent exit of gas to the exterior when the gas pressure in the exhalation path 8 distal to the exhalation valve 11 is less than or equal to the PEEP valve pressure. By selecting a PEEP valve 12 with an appropriate PEEP valve pressure it can be secured that during use of the ventilator 1 to ventilate a patient, pressure in the patient's lungs during exhalation does not fall below this PEEP valve pressure which should be at least as large as the physiological PEEP pressure, and is greater than atmospheric pressure.

A typical series of applicable passive PEEP valves would be 0 to 10 mbar, with individual passive PEEP valves within the series varying by 2.5 mbar to cover this range.

The exhalation valve 11 may be relatively simple, for example a disposable, electronically (pneumatically) or mechanically controlled valve which are embodied as modular 'plug-in' unit. The control unit 2 may operate the exhalation valve 11 either directly or indirectly.

The flow sensor 9 may be any type appropriate for measuring gas flow at the flow rates developed in the exhalation path 8, including but not limited to a flow sensor selected from the group consisting of hot film mass flow sensors, hot wire anemometers, and sensors detecting differential pressure across a low pressure drop orifice. A zero point flow sensor may be employed for measuring gas flow rates, being simpler and cheaper.

The pressure sensor 7 may be located at any site at which it is able to measure gas pressure in at least one of the following locations: the inhalation path 5, the external interface 6 and the exhalation path 8.

A most preferred embodiment of the ventilator of the invention provides the technical features of all preferred embodiments of the ventilator described above.

THE METHOD OF THE INVENTION

The method of the present invention supports or assists the patient at each breath by supplying respiratory gas at an adjustable "support pressure level". The patient breathes spontaneously; the patient triggers the start of a ventilator's inhalation phase, and may also provide the condition for terminating this inhalation phase and initiating a ventilator's exhalation phase. The method may employ the ventilator of the invention operated in pressure support ventilation mode (PSV mode). The method generates the following cycle comprising two types of phases, the ventilator inhalation phase and the ventilator exhalation phase:

    a. initiation of the ventilator exhalation phase:

- opening of exhalation valve 11,
- interrupting supply of respiratory gas;

b. detection of a condition indicating an appropriate end of the exhalation phase:

- monitoring the flow sensor for zero flow of respiratory gas, or a pre-determined flow rate approximating zero flow,
- detecting a pressure level ($PEEP_{low\_flow}$) during zero flow and determining a trigger level by subtracting a pre-determined pressure value (trigger sensitivity) from the detected pressure $PEEP_{low\_flow}$,
- monitoring pressure sensor, wherein the end of the exhalation phase is determined, when the pressure level falls below the trigger level;

c. initiation of the ventilator inhalation phase:

- closing of the exhalation valve 11,
- supply of respiratory gas to provide pressure support,

d. detection of a condition indicating an appropriate end of the inhalation phase.

- the detection of the end of the inhalation phase is preferably carried out by monitoring the flow sensor 9 for decrease of gas flow rate to a pre-determined $\dot{V}_{low\_flow}$ which is a flow rate of respiratory gas approaching zero. It is also possible to use alternative conditions for determining the end of the inhalation phase, *e.g.* the overall volume of inhaled respiratory gas or a small pressure increase due to the patient's attempt to exhale.

The following ventilator settings are selected:

(a) a maximum gas pressure value $P_{max}$ for support pressure level during inhalation phase.

(b) a gas flow rate $\dot{V}_{end\_flow}$ for respiratory gas supplied by the gas flow controller during inhalation phase at which the inhalation phase will be terminated, generally determined as a pre-determined ratio or percentage of a maximum flow rate
$\dot{V}_{peak\_flow}$ supplied by the gas flow controller during a current inhalation phase, *e.g.*
$\dot{V}_{end\_flow} = \dot{V}_{peak\_flow} \times 10\%$. Each inhalation phase is terminated when respiratory gas supplied by the gas flow controller drops to the first minimum respiratory gas flow rate $\dot{V}_{end\_flow}$.

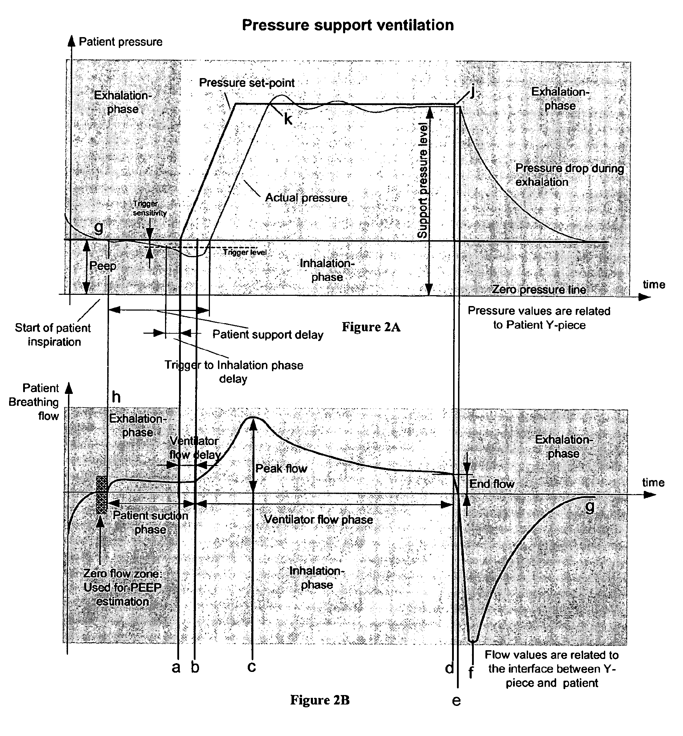

(c) a respiratory gas flow rate $\dot{V}_{low\_flow}$ for respiratory gas flowing through the exhalation path 8 during exhalation phase which approaches zero flow: the control unit treats a gas flow rate detected in the exhalation path 8 by the flow sensor 9 which is closer to zero than this respiratory gas flow rate $\dot{V}_{low\_flow}$ as being the equivalent of zero flow of gas in the exhalation path 8 ('Zero flow zone'; Fig. 2B).

It should be noted that individual inhalation and/or exhalation phases of the ventilator 1 may not correspond exactly to the respective periods of inhalation and/or exhalation of a patient using the machine.
A patient is connected via a face mask to the external connection 10 of the external interface 6.
The control unit 2 starts the ventilator inhalation phase by providing a closing signal to the exhalation valve 11 via the switch data line 13.1 and gives a delivering signal to the gas flow controller 4 via the control data line 13.2 to deliver respiratory gas to the external interface 6 via the inhalation path 5.
The initiation of delivering respiratory gas (at the external interface 6) results from patient demand for respiratory gas for a short period. This period is termed the Patient suction phase, (points h-b; Fig. 2B). The patient suction phase is the time period between the point when the patient is beginning to inhale and the point when the ventilator 1 is delivering respiratory gas. The Patient suction phase typically lasts for about 50 ms. During the patient suction phase the patient attempts to inhale which lowers the respiratory gas pressure in the ventilator 1.
During the patient suction phase and before the beginning of the inhalation phase the control unit calculates a trigger level and determines when the respiratory gas pressure decreases to this calculated trigger level. The patient suction phase also includes the termination of the exhalation phase (closing of the exhalation valve 11) by the control unit 4 and the initiation of the inhalation phase (setting of the pressure set points by the control unit 2). The time for triggering from the exhalation phase to the beginning of ventilator inhalation phase is termed the trigger to inhalation phase delay.
The patient suction phase includes a ventilator flow delay (points a-b; Fig. 2B). The ventilator flow delay is at the end of the suction phase and is the delay between beginning of ventilator inhalation phase and commencement of gas flow from the gas flow controller 4. This delay is caused due to the reaction time of control unit 2 and gas flow controller 4 and lasts typically about 2 ms. After this delay the patient suction phase ends and the gas flow controller 4 initiates the supply of respiratory gas.

At the beginning of the inhalation phase the control unit 2 sets pressure set points according to a pre-selected curve I (Fig. 2A). Curve I indicates that the control unit 2 ramps the pressure set points from an initial minimum value (beginning of inhalation phase, point a, Fig 2A) approximating PEEP to a maximum, plateau value $P_{max}$ (points i-j, Fig 2A). The value $P_{max}$ is maintained until the end of the ventilator inhalation phase (points a-j, Fig 2A). $P_{max}$ is the pressure support level that the control unit maintains during the patients inhalation phase. Shortly after the pressure set points are set to the plateau value $P_{max}$ the gas flow increases to a maximum peak flow $\dot{V}_{peak\_flow}$ (point c, Fig 2B).

The control unit 2 controls the flow of respiratory gas from the gas flow controller 4 to the external interface 6 via the inhalation path 5 according to the actual pressure of the respiratory gas (curve II, Fig. 2A).

The pressure sensor 7 measures the actual respiratory gas pressure, preferably at the external interface 6. The pressure sensor 7 transmits the value of the actual pressure to the microprocessor 3 of the control unit 2 via the pressure data line 13.3. During the inhalation phase the ventilator is controlled according to the adjusted pressure set points. The actual pressure (curve II, Fig. 2A) of respiratory gas deviates from the pressure set point due to the reaction time of the control unit. In order to achieve the optimum gas flow rate during the inhalation phase the control unit 2 is trying to adjust the actual pressure (curve II, Fig. 2A) in accordance with the pressure set points (curve I, Fig. 2A).

From the beginning of the ventilator inhalation phase to the point at which the actual pressure attains the selected maximum pressure $P_{max}$ the increase in actual pressure is delayed in comparison to the pressure set point due to factors which include time required for calculations, gas flow controller 4 reaction time, and time required for pressure to be established.

The inhalation during the patient suction phase reduces the actual pressure below PEEP valve pressure until the control unit 2 activates the gas controller 4 via the control data line 13.2 (curve II, patient suction phase, points h-b, Fig 2A). When the gas controller delivers respiratory gas then the actual pressure of respiratory gas increases from a minimum value in accordance with curve II (points b-i, Fig. 2A). The actual pressure of respiratory gas then approaches the pressure set point (point k, Fig. 2A). When the control unit 2 determines that the actual pressure is equal to the pre-selected pressure set point $P_{max}$ the control unit 2 regulates gas flow from the gas controller 4 to maintain the pressure of respiratory gas at $P_{max}$. Throughout the remainder of the ventilator inhalation phase the control unit 2 continues to adjust the gas flow from the gas flow controller 4 in accordance with the difference between the pressure set point and the actual pressure.

Due to the imperfect seal between patient and external interface a part of respiratory gas is lost.

The control unit terminates the inhalation phase when the gas flow generated by the gas flow controller 4 decreases to a selected proportion $\dot{V}_{end\_flow}$ (points d-e, Fig. 2B) of maximum gas flow ($\dot{V}_{peak\_flow}$). This condition is summarised as follows, wherein SLPM is 'standard litre per minute':

$$\text{gas flow supplied (SLPM)} < \dot{V}_{peak\_flow} \text{ (SLPM) x } \dot{V}_{end\_flow} \text{ (\% } \dot{V}_{peak\_flow} \text{ ) / 100}$$

The value of $\dot{V}_{end\_flow}$ is selected by the user. A typical value for $\dot{V}_{end\_flow}$ is 10% of $\dot{V}_{peak\_flow}$.

For example, if $\dot{V}_{peak-flow}$ is 90 SLPM, and $\dot{V}_{end\_flow}$ is 10% of $\dot{V}_{peak\_flow}$ then the ventilator 1 would enter the exhalation phase when the gas flow supplied falls below 9 SLPM.

The value selected for $\dot{V}_{end\_flow}$ ends and therefore influences the duration of the inhalation phase. Setting a value for $\dot{V}_{end\_flow}$ which is a higher percentage of $\dot{V}_{peak\_flow}$ decreases the duration of the ventilator inhalation phase, whereas setting a lower value for $\dot{V}_{end\_flow}$ increases the duration of the ventilator inhalation phase.

If a patient undergoing ventilation by the ventilator of the invention commences a spontaneous exhalation during a programmed inhalation phase, the pressure at the external interface 6 will rise for a small amount. This pressure increase will be detected by the pressure sensor 7 and sent to the control unit 2 via the pressure data line 13.3. The control unit 2 will respond by ending the programmed inhalation phase at an appropriately earlier time point. The control unit 2 will terminate the gas flow supplied by the gas flow controller 4 and initiate the next exhalation phase.

The control unit 2 terminates the ventilator's inhalation phase and commences the exhalation phase by providing an opening signal to the exhalation valve 11 via the switch data line 13.1 and by deactivating the gas flow controller 4 via the control data line 13.3, thereby stopping respiratory gas flow to the external interface 6 (point d, Fig. 2B).

At the end of the ventilator inhalation phase or the beginning of the ventilator exhalation phase, respectively, the pressure set points are reduced to the PEEP valve pressure which is maintained during the whole exhalation phase (points d-a, Fig 2A). The PEEP valve pressure is the pressure measured in the last exhalation phase. The PEEP valve secures that the pressure in the exhalation path and in the lungs of the patient will not drop below this PEEP valve pressure.

At the beginning of ventilator exhalation phase the flow rate of respiratory gas supplied by the gas controller is $\dot{V}_{end\_flow}$. Patient exhalation establishes respiratory gas flow from the external interface 6 via the exhalation path 8 and out of the ventilator 1 by passage through the following components in turn: the flow sensor 9, open exhalation valve 11, and passive PEEP valve 12.

The gas flow through the external interface 6 decreases rapidly to zero flow (point e, Fig. 2B). Zero flow is the point where the direction of the flow is changing because of the patients transfer from inhaling to exhaling.

Then the exhaled gas flow in the exhalation path 8 increases to a maximum exhaling value (point f, Fig. 2B). The amount of the exhaled gas flow then decreases towards a low flow value $\dot{V}_{low\_flow}$, at which the exhaled gas flow slowly approximates zero flow (point g, Fig. 2B). During this approach to zero flow rate the respiratory gas pressure is substantially proportional to the gas flow, due to the resistance of the exhalation path 8 and the passive PEEP valve 12. The flow sensor 9 and the exhalation valve 11 are assumed to offer essentially zero resistance (points j-g, Fig 2A or points f-g, Fig 2B, respectively).

The following parameters are used to determine the respiratory gas pressure during ventilator exhalation phase, in accordance with Formula 1:

| | |
|---|---|
| $P_{ex}$ | respiratory gas pressure during ventilator exhalation phase; |
| $PEEP_{low\_flow}$ | pressure caused by the PEEP valve 12 during ventilator exhalation phase at pre-selected low flow rate of respiratory gas; low flow being typically selected as a value of < 2SLPM; |
| $R_{PEEP\_valve}$ | resistance of PEEP valve 12 due to flow stream through narrow opening; |
| $R_{exhalation\_path}$ | resistance of all exhalation path components except PEEP valve 12; |
| $\dot{V}(t)$ | exhalation flow; |
| $\nu$ | kinematic viscosity of gas; |
| $\rho$ | specific gas density. |

## _Formula 1_

$$P_{ex} = PEEP_{low\_flow} + R_{PEEP\_valve}(\dot{V},\rho,\nu)\cdot\dot{V}(t) + R_{exhalation\_path}(\dot{V},\rho,\nu)\cdot\dot{V}(t)$$

$$P_{ex} = PEEP_{low\_flow}\Big|_{\dot{V}(t)=0}$$

As the term $R_{PEEP\_valve}$ is not accessible for a measurement the $PEEP_{low\_flow}$ can not be measured directly. It is essential to determine the $PEEP_{low\_flow}$ exactly, to detect the ideal point in time of starting the inhalation phase. A deviation from the actual $PEEP_{low\_flow}$ will shift the trigger level away from the ideal value.

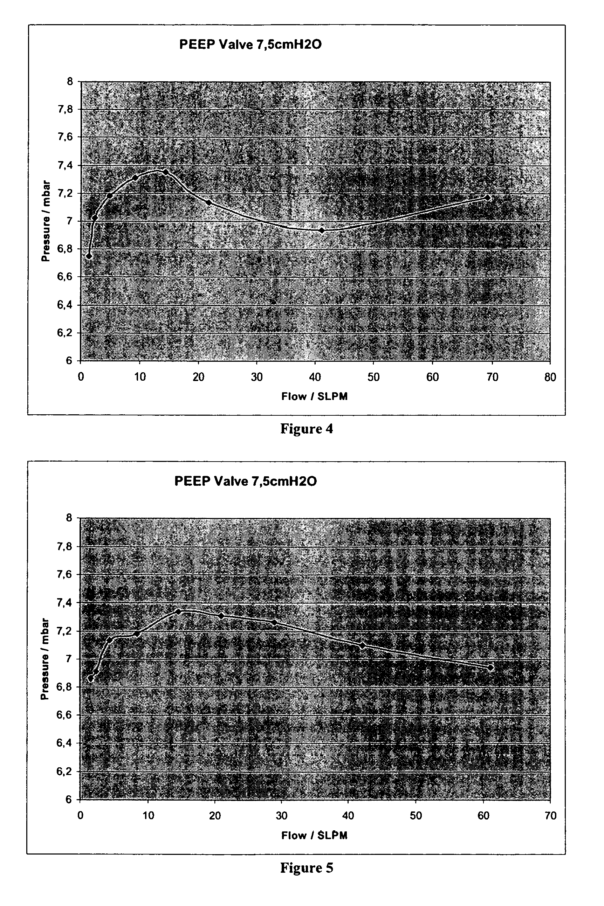

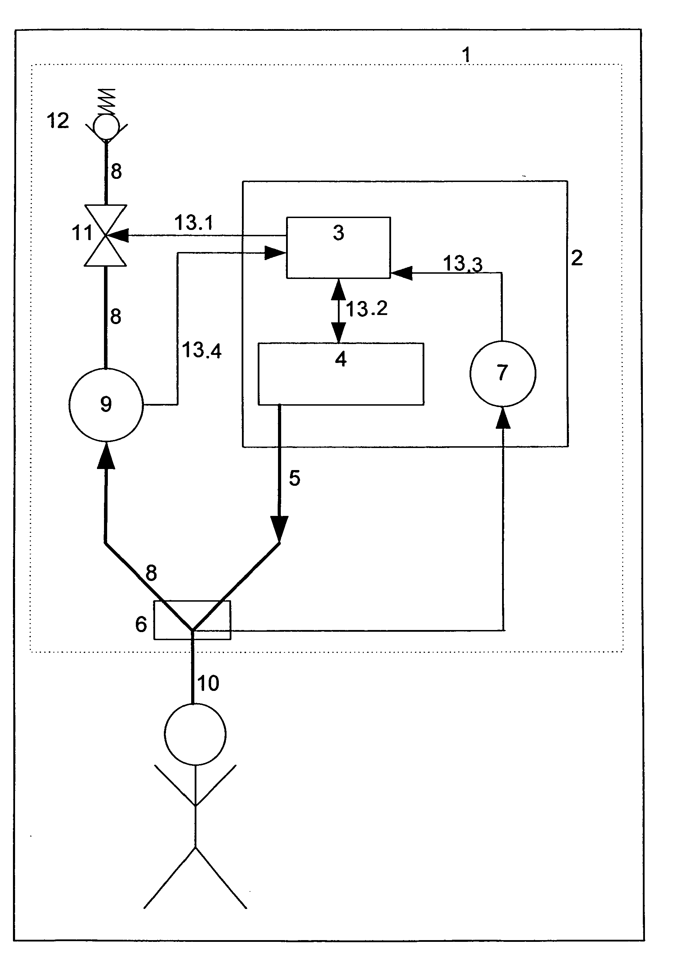

Figure 4 and 5 show flow/pressure diagrams from two different PEEP valves. Figure 4 and 5 show the value of $PEEP_{low\_flow} + R_{PEEP\_valve} * \dot{V}(t)$ as a function of flow and is not constant. Figure 4 shows, that, generally, when the flow decreases the pressure also decreases according to the flow because of the minor pressure difference. As the flow approaches zero flow there is a pressure increase because at a low flow the PEEP valve resistance increases stronger than the decrease due to the reduced flow. Therefore, there is a small pressure peak close to zero flow. Hence, it is essential to detect the zero flow phase exactly and measure the exact value of $PEEP_{low\_flow}$, The variation of the PEEP valve pressure from one exhalation phase to another may typically vary by up to 0.6 cm $H_2O$ (58,84 Pa). A Trigger sensitivity is the difference between PEEP valve pressure and trigger level within a ventilator exhalation phase. A typical trigger sensitivity is 0.4 cm $H_2O$ (39,23 Pa). In these circumstances an error in estimating PEEP valve pressure may shift the trigger level by more than the trigger sensitivity. In Figure 5 the pressure peak is extended over a larger range of flow.

When the flow rate approaches the value $\dot{V}_{low\_flow}$ which is approximately zero the second and the third term of formula 1 are also zero. Therefore the respiratory gas pressure $P_{ex}$ corresponds to the value $PEEP_{low\_flow}$ (points g-h, Fig. 2A). At $PEE_{low\_flow}$ $P_{ex}$ approximates the nominal PEEP valve pressure, which is the pressure at which the PEEP valve 12 closes (point g-h; Fig. 2A, Fig. 2B). This pressure is constant throughout the inhalation path 5, the external interface 6 and the exhalation path 8. This PEEP valve pressure will approximate the nominal PEEP value of the PEEP valve 12 selected for the patient being ventilated.

Ideally the ventilator exhalation phase terminates when respiratory gas flow through the exhalation path 8 has declined to zero $\dot{V}_{zero\_flow}$. In each ventilator exhalation phase the PEEP valve pressure would preferably be determined at zero respiratory gas flow $\dot{V}_{zero\_flow}$, because the factors influencing $P_{ex}$ are then reduced to zero in accordance with

Formula 1, and pressure $P_{ex}$ equals PEEP valve pressure.

However, during ventilator exhalation phase, the respiratory gas pressure $P_{ex}$ approaches the PEEP valve pressure over a significant time period (points g-h, Fig. 2A). Consequently PEEP valve pressure measurements are determined when respiratory gas flow has decreased to a 'low_flow'' value $\dot{V}_{low\_flow}$ rather than to the ideal 'zero_flow' $\dot{V}_{zero\_flow}$, This 'low flow' value $\dot{V}_{low\_flow}$ is a pre-selected ventilator setting. Typically the value of $\dot{V}_{low\_flow}$ for zero flow is less than 2 SLPM. Thus respiratory gas flow is substantially zero under these 'low flow' conditions, and apart from the PEEP valve pressure the factors influencing the respiratory gas pressure at this stage of ventilator exhalation phase are also substantially zero. Therefore, in accordance with Formula 1, at gas flow $\dot{V}_{low\_flow}$ the gas pressure pressure $P_{ex}$ is substantially equivalent to PEEP valve pressure, and is termed $PEEP_{low\_flow}$, The method of the present invention therefore determines for each ventilator exhalation phase a pressure $PEEP_{low\_flow}$ as the gas pressure $P_{ex}$ when gas flow is $\dot{V}_{low\_flow}$.

When the control unit 2 determines from flow meter 9 via the flow data line 13.4 that the gas flow in the exhalation path 8 has decreased to the selected 'low flow' value $\dot{V}_{low\_flow}$ the control unit 2 obtains from the pressure sensor 7 via data lines 13.3 the current respiratory gas pressure $P_{ex}$ in the exhalation path 8 and at the external interface 6. From this pressure value $P_{ex}=PEEP_{low\_flow}$ the control unit 2 calculates a trigger level $P_{trigger}$ for the current ventilator exhalation phase.

If the patient pauses after exhalation before commencing the next inhalation, the respiratory gas pressure will not fall significantly below the $PEEP_{low\_flow}$ pressure. The trigger sensitivity is determined to be adequately large to ensure that when the patient makes such a pause the respiratory gas pressure does not fall to the trigger level. Consequently the respiratory gas pressure will only fall to the trigger level if the patient commences inhalation and thereby causes a further drop in respiratory gas pressure to a pressure below that attained at the end of patient exhalation ('Patient suction phase', points h-b; Fig. 2B). The ventilating action of the ventilator is therefore co-ordinated with the requirements of the patient in that respiratory gas is provided to the patient only when the patient generates the appropriate condition for initiating ventilator inhalation phase: *i.e.* when the patient has commenced inhalation.

Then the control unit 2 terminates ventilator exhalation phase and commences the next ventilator inhalation phase when respiratory gas pressure attained in the exhalation path 8 and at the external interface 6, as measured by the pressure sensor 7, decreases to a value less than or equal to the trigger level. The Control unit 2 calculates the trigger level $T_{trigger}$ by subtracting a pre-determined difference pressure from the PEEP valve pressure $PEEP_{low\_flow}$ established as $P_{ex}$ at low flow for the current exhalation phase. This value

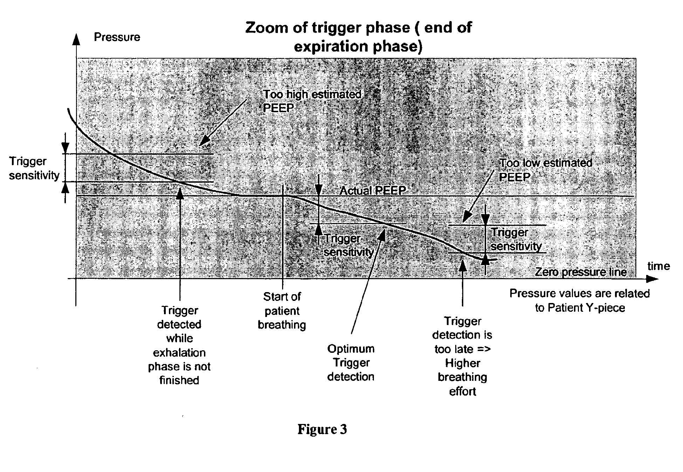

$P_{trigger}$ represents the optimum trigger level (condition II, Fig. 3). Alternatively control unit 2 may calculate the trigger level $P_{trigger}$ for the current exhalation phase as being a pre-determined proportion, *e.g.* 95%, of $PEEP_{low\_flow}$ determined for this exhalation phase.

The method of the invention then continues by cycling repetitively through the method steps defining the inhalation phase and the exhalation phase.

Fig. 3 illustrates the effect of errors in estimating PEEP valve pressure upon the timing of the transition between ventilator exhalation and inhalation phases. If $PEEP_{low\_flow}$ were to be estimated too high then termination of the ventilator exhalation phase ($P_{trigger}$) and initiation of the ventilator inhalation phase would be started too early, before ventilator exhalation phase is complete (condition I, Fig. 3). If $PEEP_{low\_flow}$ were to be estimated too low ($P_{trigger}$) then a higher 'work of breath' would be required to trigger a new ventilator inhalation phase (condition III, Fig. 3).

The method of the invention provides that the $PEEP_{low\_flow}$ will be substantially equivalent to the PEEP valve pressure even where tolerances in production of the PEEP valve result in fluctuations in PEEP valve pressure from one exhalation phase to another. Using the method of the invention control unit 2 calculates an optimum trigger level $P_{trigger}$ for each ventilator exhalation phase. Control unit 2 then determines the optimum time at which to terminate the current ventilator exhalation phase and to commence the next ventilator inhalation phase. Normally the 'work of breath' to be imposed upon a patient undergoing ventilation by the ventilator will be minimised, in order to provide the patient with maximum breathing assistance.

The method of the present invention also enables a selected 'work of breath' to be imposed upon a patient undergoing ventilation by the ventilator 1, as the 'work of breath' will vary in relation to the pre-selected trigger sensitivity (condition III, Fig. 3). An example of a situation in which such an increase in 'work of breath' may be desirable in cases where a patient is to be 'weaned off' of ventilator-assisted respiration after a prolonged period.

The advantage of the present invention is the less complex exhalation stage compared to a modern ventilator with active PEEP control and its high effort at cleaning.

Furthermore the present invention uses mainly disposable parts, which eases the handling for the operator and makes the device development inexpensive.

The PEEP valve and the exhalation valve are original equipment manufacturer products which can be bought from various manufacturers. Since the PEEP valve has no control-, energy or direct informational- path to the device the present invention is compatible to various products.

## Claims

1. A ventilator (1) suitable for assisting respiration in a mammal, said ventilator (1) comprising a control unit (2), a gas flow controller (4), an inhalation path (5) leading respiratory gas from said gas flow controller (4) to an external interface (6), an exhalation path (8) leading respiratory gas from said external interface (6) to the atmosphere, and a pressure sensor (7) for detecting the pressure of respiratory gas in the external interface (6), in the inhalation path (5) or in the exhalation path (8);
   wherein said exhalation path (8) comprises a flow sensor (9), an exhalation valve (11) and, a passive PEEP valve (12);
   wherein said ventilator (1) is embodied to operate alternately in an inhalation phase and in an exhalation phase; and
   wherein in each said exhalation phase said passive PEEP valve (12) maintains a minimum pressure in the exhalation path (8) independently of the operation of the control unit (2), and said control unit (2) is embodied to:

   - control the exhalation valve (11) open and the supply of respiratory gas from said gas flow controller (4) closed during the exhalation phase
   - measure said minimum pressure by means of the pressure sensor (7) when the absolute gas flow rate detected by the flow sensor (9) in the exhalation path (8) achieves a pre-determined minimum rate,
   - calculate a trigger level for triggering an inhalation phase, wherein said trigger level is based upon said minimum pressure, and
   - determine when the pressure detected by means of the pressure sensor (7) is less than or equal to the calculated trigger level, and trigger an inhalation phase by closing the exhalation valve (11) and supplying respiratory gas to the external interface (6) through the inhalation path (5) with the gas flow controller (4).

2. The ventilator (1) of claim 1 wherein said ventilator (1) is suitable for non-invasive assistance of respiration by pressure support ventilation.

3. The ventilator (1) of any one of claims 1 or 2, wherein said control unit (2) is embodied to calculate the trigger level by subtracting a pre-determined difference pressure from the measured minimum pressure.

4. The ventilator (1) of any one of claims 1 to 3, wherein said trigger level is calculated as being a pre-determined proportion of the measured minimum pressure.

5. The ventilator (1) of any one of claims 1 to 4, wherein said PEEP valve (12) comprises a spring-loaded membrane for controlling minimum pressure.

6. The ventilator (1) of any one of claims 1 to 5, wherein said control unit (2) operates said exhalation valve (11) indirectly.

7. The ventilator (1) of any one of claims 1 to 6, wherein said control unit (2) is embodied to terminate each inhalation phase by determining a decrease of gas flow rate to a pre-determined value.

8. The ventilator (1) of claim 7, wherein said decrease of gas flow rate is determined by variable actuation of the gas flow controller (4).

9. The ventilator (1) of claim 7, wherein said decrease of gas flow rate is determined by means of a further flow sensor located in the gas flow controller (4), in the inhalation path (5), or at the external interface (6).

10. The ventilator (1) of any one of claims 1 to 9 further comprising data lines (13) issuing from the control unit to the gas flow controller (4) and the exhalation valve (11).

11. The ventilator (1) of any one of claims 1 to 10 further comprising data lines (13) issuing from the following components to the control unit (2): the pressure sensor (7), and the gas flow sensor (9).

12. The ventilator (1) of any one of claims 1 to 11 further comprising a control line (13) issuing from the respiratory gas flow controller (4) to the control unit (2).

13. The ventilator (1) of any one of claims 10 to 12 wherein one or more of said data lines (13) operates remotely, by means of radiated energy.

14. A method of controlling the operation of a ventilator employing a passive PEEP valve (12), said ventilator (1) comprising a control unit (2), a gas flow controller (4), an inhalation path (5) leading respiratory gas from said gas flow controller (4) to an external interface (6), an exhalation path (8) leading respiratory gas from said external interface (6) to the atmosphere, and a pressure sensor (7) for detecting the pressure of respiratory gas in the external interface (6), in the inhalation path (5) or in the exhalation path (8); wherein said exhalation path (8) comprises a flow sensor (9), an exhalation valve (11) and a passive PEEP valve (12);

wherein said method comprises operating said ventilator (1) alternately in an inhalation phase and in an exhalation phase;

wherein in each said exhalation phase said passive PEEP valve (12) maintains a minimum pressure in the exhalation path (8) independently of the control of the control unit (2), the gas flow controller (4) is closed and the exhalation valve (11) is open; and

wherein, in each said exhalation phase, said method comprises the steps of:

    a. initiation of the ventilator exhalation phase:

        - opening of exhalation valve (11),
        - interrupting supply of respiratory gas;

    b. detection of a condition indicating an appropriate end of the exhalation phase:

        - monitoring the flow sensor for zero flow of respiratory gas, or a predetermined flow rate approximating zero flow,
        - detecting a pressure level ($PEEP_{low\_flow}$) during zero flow and determining a trigger level by subtracting a pre-determined pressure value (trigger sensitivity) from the detected pressure $PEEP_{low\_flow}$,
        - monitoring pressure sensor, wherein the end of the exhalation phase is determined, when the pressure level falls below the trigger level;

    c. initiation of the ventilator inhalation phase:

        - closing of the exhalation valve (11),
        - supply of respiratory gas to provide pressure support,

    d. detection of a condition indicating an appropriate end of the inhalation phase.

        - the detection of the end of the inhalation phase is preferably carried out by monitoring the flow sensor (9) for decrease of gas flow rate to a pre-determined $\dot{V}_{low\_flow}$ which is a flow rate of respiratory gas approaching zero.

15. The method of claim 14, further comprising the steps e. and f.:

    e. operating said ventilator (1) in inhalation phase until said inhalation phase is terminated and a new exhalation phase is commenced, and
    f. continuing to operate said ventilator (1) by cycling repetitively through steps a. to f.

16. The method of claims 14 to 15, further comprising, within step e., the steps of:

    e(i) operating said ventilator (1) in said inhalation phase with said gas flow controller (3) supplying respiratory gas at a progressively decreasing flow rate as the pressure of respiratory gas at the external interface (6) approaches a pre-determined maximum pressure, and
    e(ii) terminating said inhalation phase and commencing said new exhalation phase when the flow of respiratory gas supplied by the gas flow controller (3) is less than or equal to a pre-determined minimum value.

17. The method of any one of claims 14 to 16, wherein said trigger level is calculated by subtracting a pre-determined difference pressure from the measured minimum pressure.

**18.** The method of any one of claims 14 to 17, wherein said trigger level is calculated as being a pre-determined proportion of the measured minimum pressure.

**19.** The method of any one of claims 14 to 18, wherein said control unit (2) operates said exhalation valve (11) indirectly.

**20.** The method of any one of claims 14 to 19, wherein each inhalation phase is terminated by determining a decrease of gas flow rate to a pre-determined value.

**21.** The method of claim 20 wherein said decrease of gas flow rate is determined by variable actuation of the gas flow controller (4).

**22.** The method of claim 20 wherein said decrease of gas flow rate is determined by means of a separate flow meter located in the gas flow controller (4), in the inhalation path (5), or at the external interface (6).

**23.** The method of any one of claims 14 to 22, wherein said ventilator (1) is the ventilator of any one of claims 1 to 13.

**24.** The method of any one of claims 14 to 23, wherein the detection of the end of the inhalation phase is preferably carried out by monitoring the flow sensor (9) for decrease of gas flow rate to a predetermined $\dot{V}_{low\_flow}$ which is a flow rate of respiratory gas approaching zero.

Figure 1

## Pressure support ventilation

Figure 2A

Figure 2B

Figure 3

Figure 4

Figure 5

**European Patent Office**

**PARTIAL EUROPEAN SEARCH REPORT**     **Application Number**

which under Rule 63 of the European Patent Convention EP 08 00 6506
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 691 134 A (TEIJIN LTD [JP]) 10 January 1996 (1996-01-10) * page 4, line 28 - page 5, line 58; figure 2 * ----- | 1-13 | INV. A61M16/00 A61M16/20 |
| A | US 4 257 453 A (KOHNKE OLE B) 24 March 1981 (1981-03-24) * column 1, line 5 - line 30; figure 1 * ----- | 1,5 | |
| X | WO 96/40337 A (NELLCOR PURITAN BENNETT INC [US]) 19 December 1996 (1996-12-19) * page 6, line 8 - page 7, line 30 * ----- | 1-13 | |
| A | US 3 933 171 A (HAY WAYNE W) 20 January 1976 (1976-01-20) * column 4, line 1 - line 40 * ----- | 1,5 | |
| X | EP 1 795 222 A (GEN ELECTRIC [US]) 13 June 2007 (2007-06-13) * paragraph [0023] - paragraph [0024] * ----- -/-- | 1-4, 10-13 | |

**TECHNICAL FIELDS SEARCHED (IPC)**

A61M

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 9 September 2008 | Kroeders, Marleen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

**European Patent Office**

**PARTIAL EUROPEAN SEARCH REPORT**

Application Number

EP 08 00 6506

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | EP 0 982 043 A (SIEMENS ELEMA AB [SE] MAQUET CRITICAL CARE AB [SE]) 1 March 2000 (2000-03-01) * paragraph [0026] - paragraph [0028] * ----- | 1-4, 10-13 | |
| X | WO 02/32488 A (NEWPORT MEDICAL INSTR INC [US]; HONG LIN DU [US] NEWPORT MEDICAL INSTR) 25 April 2002 (2002-04-25) * page 5, line 33 - page 6, line 25 * ----- | 1-4, 10-13 | |

TECHNICAL FIELDS SEARCHED (IPC)

EPO FORM 1503 03.82 (P04C10)

**INCOMPLETE SEARCH
SHEET C**

```
Claim(s) not searched:
        14-24

Reason for the limitation of the search (non-patentable invention(s)):

Article 53 (c) EPC - Method for treatment of the human or animal body by
therapy
```

**ANNEX TO THE EUROPEAN SEARCH REPORT
ON EUROPEAN PATENT APPLICATION NO.**                    EP 08 00 6506

This annex lists the patent family members relating to the patent documents cited in the above-mentioned European search report.
The members are as contained in the European Patent Office EDP file on
The European Patent Office is in no way liable for these particulars which are merely given for the purpose of information.

09-09-2008

| Patent document cited in search report | | Publication date | Patent family member(s) | | Publication date |
|---|---|---|---|---|---|
| EP 0691134 | A | 10-01-1996 | AU<br>AU<br>US | 683753 B2<br>2484595 A<br>5572993 A | 20-11-1997<br>18-01-1996<br>12-11-1996 |
| US 4257453 | A | 24-03-1981 | NONE | | |
| WO 9640337 | A | 19-12-1996 | US | 6041780 A | 28-03-2000 |
| US 3933171 | A | 20-01-1976 | NONE | | |
| EP 1795222 | A | 13-06-2007 | US | 2007125377 A1 | 07-06-2007 |
| EP 0982043 | A | 01-03-2000 | DE<br>JP<br>US | 69936170 T2<br>2000070370 A<br>6679258 B1 | 10-04-2008<br>07-03-2000<br>20-01-2004 |
| WO 0232488 | A | 25-04-2002 | AT<br>AU<br>DE<br>DE<br>EP<br>US | 290900 T<br>1322102 A<br>60109474 D1<br>60109474 T2<br>1326670 A2<br>6622726 B1 | 15-04-2005<br>29-04-2002<br>21-04-2005<br>13-04-2006<br>16-07-2003<br>23-09-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020088465 A1, Hill
- US 20020023645 A1, Zdrojkowski
- US 4211221 A
- EP 0811394 A1, Christian

**Non-patent literature cited in the description**

- **Hotchkiss et al.** *Am J Respir Crit Care Med,* 2002, vol. 165, 47-53